# EUROPEAN PATENT SPECIFICATION

(11) **EP 3 745 975 B1**
(45) Date of publication and mention of the grant of the patent: **03.07.2024**
(21) Application number: 19746924.0
(22) Date of filing: 01.02.2019
(51) Int. Cl.: A61B 18/18, A61B 17/32, A61B 18/12, A61B 18/14

(54) **MICROWAVE ABLATION ANTENNAS**
MIKROWELLENABLATIONSANTENNEN
ANTENNES D'ABLATION PAR MICRO-ONDES

(30) Priority: 02.02.2018 US 201862625840 P; 19.03.2018 US 201862644989 P; 06.06.2018 US 201862681518 P
(43) Date of publication of application: 09.12.2020
(73) Proprietor: Varian Medical Systems, Inc., Palo Alto, CA 94304 (US)
(72) Inventor: NEBRIGIC, Dragan D., Austin, Texas 78717 (US); DILLON, James L., Austin, Texas 78717 (US); DESMIT, Daniel Anthony, Austin, Texas 78717 (US); WEINBERG, Samuel, Austin, Texas 78717 (US)
(74) Representative: Foster, Mark Charles
(86) International application number: PCT/US2019/016375
(87) International publication number: WO 2019/152856

(56) References cited:
- WO-A1-98/49933
- CN-A- 105 769 338
- US-A- 4 399 419
- US-A- 4 700 716
- US-A1- 2006 289 528
- US-A1- 2011 118 723
- US-A1- 2011 118 724
- US-A1- 2013 072 924
- US-A1- 2013 267 940
- US-A1- 2015 223 884
- US-A1- 2016 278 856
- US-A1- 2017 172 656
- US-A1- 2017 231 696

## Description

### BACKGROUND

### 1. Field

Embodiments of the invention generally relate to electromagnetic radiation (EMR) therapy and more particularly to applicators and systems for applying electromagnetic energy to a treatment site to heat tissue needing treatment at the treatment site. Embodiments of the invention are useful particularly for treatments in the nature of microwave coagulation or ablation.

### 2. Description of the Related Art

The use of electromagnetic (EM) energy to heat tissue for the treatment of disease is known. In using microwave energy for tissue heating, an applicator having a microwave radiating antenna is positioned with respect to the tissue to be treated (heated) so that microwave energy radiated from the antenna penetrates and heats/ablates the tissue. Many microwave applicators are known in the art. Death, or necrosis, of living tissue cells occurs at temperatures elevated above a normal cell temperature for a sufficient period of time. The sufficient period of time is generally dependent upon the temperature to which the cells are heated. Above a threshold temperature of about 41.5° C, substantial thermal damage occurs in most malignant cells. At temperatures above about 45° C, thermal damage occurs to most normal cells. During treatment, it is desirable to produce an elevated temperature within the target tissue for a time period sufficient to cause the desired cell damage, while keeping nearby healthy tissue at a safe, lower temperature. For this reason, when treatment involving tissue heating is used, it is important to assure both (i) adequate tumor/diseased tissue heating throughout the tumor/diseased to the tumor/diseased tissue margin and (ii) reduced temperatures in the normal/undiseased tissue surrounding the tumor/diseased tissue.

Heating therapy is sometimes combined with other treatments, such as surgery, ionizing radiation, and chemotherapy. For example, when heating is combined with radiation, it is desirable to maintain the temperature within the diseased tissue within the range of about 42° C to 45° C. Higher temperatures are usually undesirable when a combined treatment modality is used because higher temperatures can lead to microvessal collapse causing resistance to other therapies such as, radiation therapy and chemotherapy, and decrease the amount of systemic chemotherapy from reaching the tumor as a result of vascular damage. Lower temperatures are undesirable because these temperatures can fail to provide adequate heating and thereby, reduce the therapeutic effect of the treatment. Therefore, it is important to control the temperature within the desired range for multi-modality treatments and not allow heating of the tissue in the tumor/diseased tissue or surrounding the tumor to temperatures above about 45° C because heating the tissue to these temperatures may damage the tissue, compromising the effectiveness of other treatments. Treatment within this controlled temperature range is usually referred to as hyperthermia.

Forms of thermal therapy that kill the tissue with heating alone are generally referred to as coagulation or ablation. To adequately eradicate a cancerous tumor/diseased tissue with only the application of heat, it is necessary to ensure sufficient heating is accomplished throughout the entire tumor/tissue. In cases of a malignant tumor, if viable tumor cells are left behind, the tumor can rapidly grow back leaving the patient with the original problem. In what is generally referred to as microwave coagulation or microwave ablation, the diseased tissue is heated to at least about 55° C, and typically to above about 60° C, for exposure times sufficient to kill the cells, typically, for greater than about one minute. With microwave coagulation and ablation treatments, there is a volume reduction of temperature that ranges from the high temperature in the treated tissue to the normal tissue temperature of 37° C outside the treated tissue. The outer margin of the overall heat distribution in the treated tissue volume may then result in damage to normal tissue if such normal tissue is overheated. Therefore, for prolonged coagulation or ablation treatments where the coagulation or ablation volume is maintained at very high temperatures, there is a high risk of damage to the surrounding, normal tissues.

For proper treatment of targeted cancerous tumor volumes or other tissue volumes to be treated, it becomes very important to properly deliver the correct, targeted thermal distribution over a sufficient time period to eradicate the tumor tissue while minimizing damage to critical surrounding normal tissue. Fortunately, there are tumor locations that reside in normal tissue that can be destroyed by the heating in limited areas without affecting the health of the patient, such as liver tissue. In such situations the coagulation can be applied in an aggressive way to include a margin of safety in destruction of limited surrounding normal tissues to assure that all of the cancerous tumor/diseased tissue is destroyed.

The process of heating very rapidly to high temperatures that is common in coagulation and ablation treatments may utilize a rather short exposure time. In doing so, the resulting temperature distribution becomes primarily a result of the power absorption distribution within the tissue. However, if such treatments continue for multiple minutes, the blood flow and thermal conduction of the tumor/diseased tissue and surrounding tissues will modify the temperature distribution to result in a less predictable heat distribution because the changes occurring in blood flow in such a heated region, may not be predictable. Therefore, it is important to optimize the uniformity of the tissue heating power that is absorbed to lead to a more predictable temperature distribution that better corresponds with the treatment prescription. Therefore, pretreatment planning practices prior to and possibly during treatment for calculating the power and temperature distribution resulting from the parameters of power and relative phase of the power applied to the tissue can be important for not only coagulation and ablation, but also hyperthermia. As higher temperatures are used during treatment, it may increase patient discomfort and pain, so it can be helpful to avoid excessive temperatures to reduce the need of patient sedation.

Invasive microwave energy applicators can be inserted into living body tissue to place the source of heating into or adjacent to a diseased tissue area. Invasive applicators help to overcome some difficulties that surface applicators experience when the target tissue region is located below the skin (e.g., the prostrate). Invasive applicators must be properly placed to localize the heating to the vicinity of the desired treatment area. Even when properly placed, however, it has been difficult to ensure that adequate heat is developed in the diseased tissue without overheating surrounding healthy tissue. Further, with applicators operating at higher power levels to produce the needed higher temperatures for coagulation and ablation, there is a tendency for the coaxial cable in the portion of the applicator leading from outside the body to the location of the radiating antenna in the applicator to heat to undesirably high temperatures, which can cause thermal damage to the normal tissue through which the applicator passes to reach the diseased tissue to be treated. Therefore, various ways of cooling the applicator have been used with prior devices.

While many microwave applicators are known in the art for applying microwave energy to tissue to provide heating to the tissue, inter alia those disclosed in US2017/0172656 A1, there is a need for better applicators that are easy to use, that have more consistent and predictable spherical heating patterns that can be better tuned in order to perform more efficient and consistent ablations.

### SUMMARY

According to a first aspect of the invention, there is provided an elongate applicator body for use with a microwave applicator, in accordance with claim 1.

According to a second aspect of the invention, there is provided a microwave applicator for insertion into body tissue, in accordance with claim 13.

According to a third aspect of the invention, there is provided a microwave applicator for insertion into body tissue, in accordance with claim 17.

Optional features are defined in the dependent claims.

Embodiments are directed to an elongate applicator body for use with a microwave applicator where the elongate applicator body comprises a tip portion, a first dielectric isolator having a first dielectric width (LD1), a first shunt, a second dielectric isolator having a second dielectric width (LD2), a second shunt, a third dielectric isolator having a third dielectric width (LD3), a microwave transmission line and an outer sleeve.

Embodiments are also directed to an elongate applicator body for use with a microwave applicator where the elongate applicator body comprises a tip portion having trocar tip and an elongate portion, a first dielectric isolator having a first cylindrical portion with a first isolator diameter and a first isolator width (LD1) and an elongated second cylindrical portion with a second portion length and a first isolator second diameter that is less than the first isolator diameter, a first cylindrical shunt having a first shunt diameter and a first shunt width, a second cylindrical dielectric isolator having a second isolator diameter and second isolator width(LD2), a second cylindrical shunt having a second shunt diameter and a second shunt width, a third cylindrical dielectric isolator having a third isolator diameter and a third isolator width (LD3), a microwave transmission line and an outer sleeve.

Further embodiments are also directed to an elongate applicator body for use with a microwave applicator. In some embodiments, the elongate applicator body comprises a tip portion having trocar tip and an elongate portion, a first dielectric isolator having a first cylindrical portion with a first isolator diameter and a first isolator width (LD1) and an elongated second cylindrical portion with a second portion length and a first isolator second diameter that is less than the first isolator diameter, a first cylindrical shunt having a first shunt diameter and a first shunt width, a second cylindrical dielectric isolator having a second isolator diameter and second isolator width (LD2), a second cylindrical shunt having a second shunt diameter and a second shunt width, a third cylindrical dielectric isolator having a third isolator diameter and a third isolator width (LD3), a microwave transmission line, an outer sleeve and a magnetic sleeve.

Embodiments are also directed to an elongate applicator body for use with a microwave applicator where the elongate applicator body comprises a tip portion having trocar tip and a tip elongate portion, a first dielectric isolator having a first cylindrical portion with a first isolator diameter and a first isolator width (LD1) and an elongated second cylindrical portion with a second portion length and a first isolator second diameter that is less than the first isolator diameter, a first cylindrical shunt having a first shunt diameter and a first shunt width, a second cylindrical dielectric isolator having a second isolator diameter and second isolator width (LD2), a second cylindrical shunt having a second shunt diameter and a second shunt width, a third cylindrical dielectric isolator having a third isolator diameter and a third isolator width (LD3) and a microwave transmission line. Additionally, the first cylindrical shunt, the second cylindrical dielectric isolator, the second cylindrical shunt and the third cylindrical dielectric isolator are disposed on the elongated second cylindrical portion of the first dielectric isolator.

Further embodiments are directed to an elongate applicator body for use with a microwave applicator. The elongate applicator body comprises a tip portion having trocar tip and a tip elongate portion, a first dielectric isolator having a first cylindrical portion with a first isolator diameter and a first isolator width (LD1) and an elongated second cylindrical portion with a second portion length and a first isolator second diameter that is less than the first isolator diameter, a first cylindrical shunt having a first shunt diameter and a first shunt width, a second cylindrical dielectric isolator having a second isolator diameter and second isolator width (LD2), a second cylindrical shunt having a second shunt diameter and a second shunt width, a third cylindrical dielectric isolator having a third isolator diameter and a third isolator width (LD3) and a microwave transmission line. Additionally, the first cylindrical shunt, the second cylindrical dielectric isolator, the second cylindrical shunt and the third cylindrical dielectric isolator are disposed on the elongated second cylindrical portion of the first dielectric isolator. Further, (i) at least a portion of the tip elongate portion is disposed within a longitudinal lumen of the first dielectric isolator, (ii) at least a portion of the microwave transmission line is also disposed within a portion of the longitudinal lumen of the first dielectric isolator, (iii) an inner conductor of the microwave transmission line is received within and end of the tip elongate portion, (iv) the first cylindrical portion of the first cylindrical dielectric isolator is adjacent to the trocar tip, (v) the first cylindrical shunt is adjacent to the first cylindrical portion, (vi) the second cylindrical dielectric isolator is adjacent to the first cylindrical shunt, (vii) the second cylindrical shunt is adjacent to the second cylindrical dielectric isolator and (viii) the third cylindrical dielectric isolator is adjacent to the second cylindrical shunt.

Embodiments are also directed to a microwave applicator for insertion into body tissue where the microwave applicator comprises an elongate applicator body having an insertion end for insertion into a tissue region of the living body and an attachment end for attachment to a source of microwave energy, a tip portion, a first dielectric isolator having a first dielectric width (LD1), a first shunt, a second dielectric isolator having a second dielectric width (LD2), a second shunt and a third dielectric isolator having a third dielectric width (LD3). The microwave applicator further includes a microwave energy transmission line disposed within the elongate applicator body to conduct microwave energy from the attachment end of the applicator to the insertion end, the microwave energy transmission line having an inner conductor and an outer conductor, an outer sleeve extending around and spaced from the outer conductor of the microwave energy transmission line to form an outside of a portion of the elongate applicator body and to provide a cooling fluid space between the outer conductor of the microwave transmission line and an inside surface of the outer sleeve, and a guide sleeve positioned concentrically around and spaced from the outer conductor of the microwave energy transmission line and the inside surface of the outer sleeve to divide at least a portion of the cooling fluid space into an inner cooling fluid space along the outer conductor of the microwave energy transmission line and an outer cooling fluid space along the inside surface of the outer sleeve, the inner cooling fluid space communicating with the outer cooling fluid space wherein the guide sleeve is adapted to guide flow of a cooling fluid within the cooling fluid space to cool the microwave energy transmission line and the outer sleeve.

Embodiments are also directed to a microwave applicator for insertion into body tissue. In some embodiments, the microwave applicator comprises an elongate applicator body including a tip portion having a trocar tip, a first dielectric isolator having a first cylindrical portion with a first isolator diameter and a first isolator width (LD1) and an elongated second cylindrical portion with a second portion length and a first isolator second diameter that is less than the first isolator diameter, a first cylindrical shunt having a first shunt diameter and a first shunt width, a second cylindrical dielectric isolator having a second isolator diameter and second isolator width (LD2), a second cylindrical shunt having a second shunt diameter and a second shunt width, and a third cylindrical dielectric isolator having a third isolator diameter and a third isolator width (LD3). The microwave applicator also includes a microwave energy transmission line disposed within the elongate applicator body to conduct microwave energy from the attachment end of the applicator to the insertion end, the microwave energy transmission line having an inner conductor and an outer conductor, an outer sleeve extending around and spaced from the outer conductor of the microwave energy transmission line to form an outside of a portion of the elongate applicator body and to provide a cooling fluid space between the outer conductor of the microwave transmission line and an inside surface of the outer sleeve, and a guide sleeve positioned concentrically around and spaced from the outer conductor of the microwave energy transmission line and the inside surface of the outer sleeve to divide at least a portion of the cooling fluid space into an inner cooling fluid space along the outer conductor of the microwave energy transmission line and an outer cooling fluid space along the inside surface of the outer sleeve, the inner cooling fluid space communicating with the outer cooling fluid space wherein the guide sleeve is adapted to guide flow of a cooling fluid within the cooling fluid space to cool the microwave energy transmission line and the outer sleeve.

Further embodiments are also directed to a microwave applicator for insertion into body tissue where the microwave applicator comprises an elongate applicator body having an insertion end for insertion into a tissue region of the living body and an attachment end for attachment to a source of microwave energy. In some embodiments, the elongate applicator body comprises a tip portion having trocar tip and an elongate portion, a first dielectric isolator having a first cylindrical portion with a first isolator diameter and a first isolator width (LD1) and an elongated second cylindrical portion with a second portion length and a first isolator second diameter that is less than the first isolator diameter, a first cylindrical shunt having a first shunt diameter and a first shunt width, a second cylindrical dielectric isolator having a second isolator diameter and second isolator width (LD2), a second cylindrical shunt having a second shunt diameter and a second shunt width, and a third cylindrical dielectric isolator having a third isolator diameter and a third isolator width (LD3), wherein the first cylindrical shunt, the second cylindrical dielectric isolator, the second cylindrical shunt and the third cylindrical dielectric isolator are disposed on the elongated second cylindrical portion of the first dielectric isolator. The microwave applicator further includes a microwave energy transmission line disposed within the elongate applicator body to conduct microwave energy from the attachment end of the applicator to the insertion end, the microwave energy transmission line having an inner conductor and an outer conductor, an outer sleeve extending around and spaced from the outer conductor of the microwave energy transmission line to form an outside of a portion of the elongate applicator body and to provide a cooling fluid space between the outer conductor of the microwave transmission line and an inside surface of the outer sleeve, and a guide sleeve positioned concentrically around and spaced from the outer conductor of the microwave energy transmission line and the inside surface of the outer sleeve to divide at least a portion of the cooling fluid space into an inner cooling fluid space along the outer conductor of the microwave energy transmission line and an outer cooling fluid space along the inside surface of the outer sleeve, the inner cooling fluid space communicating with the outer cooling fluid space wherein the guide sleeve is adapted to guide flow of a cooling fluid within the cooling fluid space to cool the microwave energy transmission line and the outer sleeve.

Further embodiments are directed to an elongate applicator body for use with a microwave applicator where the elongate applicator body comprises a tip portion having trocar tip and a tip elongate portion, a dielectric isolator having a first major cylindrical portion with a first major isolator diameter and a first major isolator width (LD1), a first minor cylindrical portion with a first minor isolator diameter less than the first major isolator diameter and a first minor isolator width, a second major cylindrical portion with a second major isolator diameter and a second major isolator width (LD2), a second minor cylindrical portion with a second minor isolator diameter less than the second major isolator diameter and a second minor isolator width, and a third major cylindrical portion with a third major isolator diameter greater than the second minor isolator diameter and a third major isolator width (LD3). The elongate applicator body also includes a first cylindrical shunt having a first shunt diameter and a first shunt width, where the first cylindrical shunt is disposed on the first minor cylindrical portion, a second cylindrical shunt having a second shunt diameter and a second shunt width, where the second cylindrical shunt is disposed on the second minor cylindrical portion, a microwave transmission line, and an outer sleeve.

The description, objects and advantages of embodiments of the present invention will become apparent from the detailed description to follow, together with the accompanying drawings.

### BRIEF DESCRIPTION OF THE DRAWINGS

The above-mentioned aspects, as well as other features, aspects and advantages of the present technology will now be described in connection with various embodiments, with reference to the accompanying drawings. The illustrated embodiments, however, are merely examples and are not intended to be limiting. Throughout the drawings, similar symbols typically identify similar components, unless context dictates otherwise. Note that the relative dimensions of the following FIGS. may not be drawn to scale.
FIG. 1 is a side elevation of an applicator, according to an embodiment of the present invention;
FIG. 2 is a cross-sectional view of a portion of the applicator depicted in FIG. 1;
FIG. 3 is a cut away perspective view of a portion of the applicator depicted in FIG. 2
FIG. 4 is a side elevation of an applicator, according to an embodiment of the present invention;
FIG. 5 is a side view of an elongate applicator body, according to an embodiment of the invention;
FIG. 6 is an exploded side view of the elongate applicator body depicted in FIG. 5;
FIG. 7 is an exploded view of a microwave antenna portion of an elongate applicator body, according to an embodiment of the invention;
FIG. 8 is a side view of the assembled microwave antenna portion depicted in FIG. 7;
FIG. 9A is a side view of a first dielectric isolator, according to an embodiment of the invention;
FIG. 9B is a longitudinal view of the first dielectric isolator depicted in FIG. 9A;
FIG. 9C is a cross-sectional view taken along line A-A in FIG. 9B;
FIG. 10 is a perspective view of a second dielectric isolator, according to an embodiment of the invention;
FIG. 11 is a perspective view of a third dielectric isolator, according to an embodiment of the invention;
FIG. 12 is a side view of a coaxial microwave transmission line, according to an embodiment of the invention;
FIG. 13 is an exploded view of a microwave antenna portion of an elongate applicator body, according to an embodiment of the invention;
FIG. 14 is a cross-sectional of the assembled microwave antenna portion depicted in FIG. 13;
FIG. 15 is a temperature profile of the microwave energy field of an applicator, according to an embodiment of the invention;
FIG. 16 is a temperature profile of the microwave energy field of an applicator, according to an embodiment of the invention;
FIG. 17 is a temperature profile of the microwave energy field of an applicator, according to an embodiment of the invention;
FIG. 18 is a side view of an assembled microwave antenna portion, according to an embodiment of the invention;
FIG. 19 is a temperature profile of the microwave energy field of an applicator, according to an embodiment of the invention; and
FIG. 20 is a temperature profile of the microwave energy field of an applicator, according to an embodiment of the invention.

### DETAILED DESCRIPTION

While methods may be depicted in the drawings or described in the specification in a particular order, such methods need not be performed in the particular order shown or in sequential order, and that all methods need not be performed, to achieve desirable results. Other methods that are not depicted or described can be incorporated in the example methods and processes. For example, one or more additional methods can be performed before, after, simultaneously, or between any of the described methods. Further, the methods may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Reference to a singular item, includes the possibility that there are plural of the same items present. More specifically, as used herein and in the appended claims, the singular forms "a," "an," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It will be understood that when an element is referred to as being "connected" or "coupled" to another element, it can be directly connected or coupled to the other element or intervening elements may be present. In contrast, if an element is referred to as being "directly connected" or "directly coupled" to another element, there are no intervening elements present.

It will also be understood that, although the terms first, second, etc. may be used herein to describe various elements, these elements should not be limited by these terms. These terms are only used to distinguish one element from another. Thus, a first element could be termed a second element without departing from the teachings of the present invention.

Language of degree used herein, such as the terms "approximately," "about," "generally," and "substantially," represent a value, amount, or characteristic close to the stated value, amount, or characteristic that still performs a desired function or achieves a desired result. For example, the terms "approximately," "about," "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. Additionally, numeric ranges are inclusive of the numbers defining the range, and any individual value provided herein can serve as an endpoint for a range that includes other individual values provided herein. For example, a set of values such as 1, 2, 3, 8, 9, and 10 is also a disclosure of a range of numbers from 1-10, from 1-8, from 3-9, and so forth.

Some embodiments have been described in connection with the accompanying drawings. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

While a number of embodiments and variations thereof have been described in detail, other modifications and methods of using the same will be apparent to those of skill in the art.

Thermal ablation may be used to treat many conditions and diseases including, and not limited to, cancerous tissue. These conditions and diseases can be treated in many organs of the human body including, and not limited to, the liver, lungs, kidneys, prostate, bladder, ovaries, cervix, uterus, endometrium, breasts, brain, stomach, colon and skin. In treating certain conditions, it is imperative to ensure ablation of the diseased tissue while not damaging healthy tissue adjacent to the ablation site. Accordingly, embodiments of the present invention are directed to microwave applicator devices that have a more predictable and controlled ablation zone.

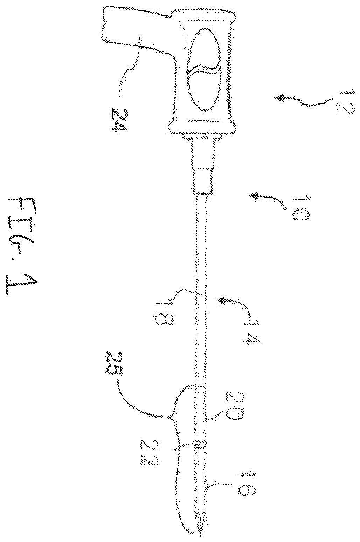

An embodiment of a microwave applicator for microwave coagulation and ablation treatment of diseased tissue within living body tissue is illustrated in FIG. 1. The applicator, referred to generally as 10, includes a handle 12 from which a substantially rigid elongate applicator body 14 extends with an insertion tip 16 forming the insertion end portion of the applicator for insertion into a region of the target tissue to be ablated. The substantially rigid elongate applicator body 14 includes an outer conductive sleeve 18 extending from the handle 12, at least one conductive shunt 20, the conductive insertion tip 16, and at least one dielectric collar/isolator 22 positioned between the insertion tip 16 and the shunt 20. The outside diameters of the exposed portions of the outer conductive sleeve 18, the conductive shunt 20, the dielectric collar 22, and the insertion tip 16 (which may be sharpened at its insertion end 17), are all about equal so as to form a smooth continuous elongate applicator body for insertion into the target tissue. The elongate applicator body may be coated with a stick resistant dielectric material such as Teflon, not shown. A pistol grip 24 allows the handle 12 to be easily held for manipulation of the applicator.

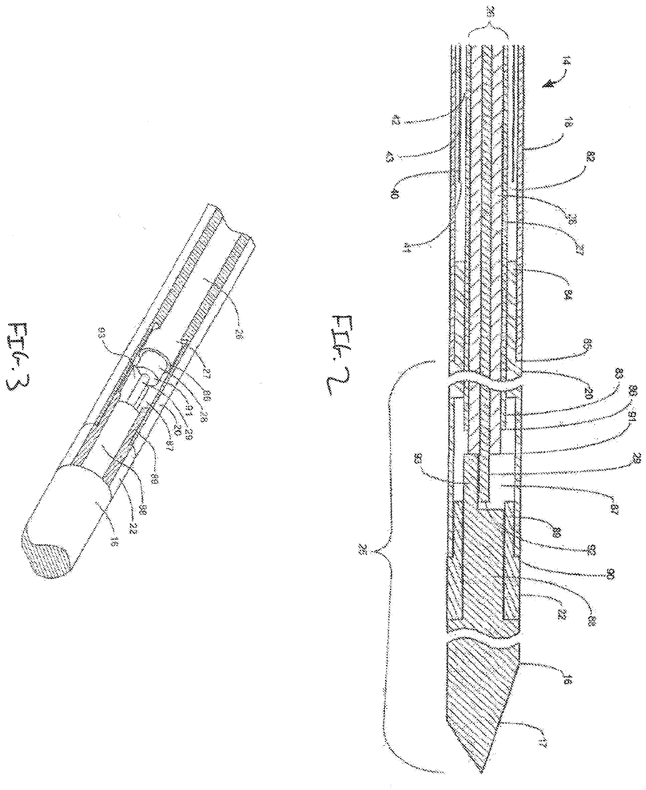

The applicator 10 has a microwave antenna portion 25 toward the insertion tip 16 of the elongate applicator body 14 to radiate microwave energy from the antenna portion 25 into the target tissue. Microwave energy is transmitted from the handle 12 through the elongate applicator body to the antenna portion 25 by a coaxial microwave transmission line 26 (as depicted in FIGS. 2 and 3), within the elongate applicator body 14 and having an inner conductor 29 and an outer conductor 27 separated by a dielectric material/isolator 28 positioned therebetween. Although not required, the coaxial transmission line 26 may be a semirigid coaxial cable with copper inner and outer conductors and a Teflon or Teflon and air dielectric material. No outer dielectric insulating material is used. Such coaxial cable will usually have about a fifty ohm impedance, which provides a good impedance match to the microwave generator and to typical living body tissue characteristics.

The outer diameter of the coaxial transmission line 26 (also the outer diameter of the outer conductor 27 of the coaxial transmission line) is smaller than the inside diameter of the outer conductive sleeve 18 so a space 82 is provided between the transmission line and the outer conductive sleeve. This space 82 will be referred to as a cooling fluid space. Conductive shunt 20 is positioned around and in electrical contact with both the insertion end portion 83 of the transmission line outer conductor 27, and the outer conductive sleeve 18. Shunt 20 includes a reduced outer diameter end portion 84 toward the handle end of the applicator dimensioned to fit into the space 82 between the outside surface of the outer conductor 27 of the coaxial transmission line 26 and the inside surface of the outer conductive sleeve 18. Shunt 20 can be soldered to both the outer conductor 27 and the outer sleeve 18 to ensure good electrical connection. Soldering will also secure shunt 20 to outer sleeve 18 for a strong connection of shunt 20 to sleeve 18. However, shunt 20 can be secured to sleeve 18 and, if desired, to outer conductor 27 by a bonding agent, such as, for example, an epoxy adhesive material. As will be understood by those of skill in the art, other bonding materials may be used. If the bonding agent is conductive, it can replace soldering. With this connection, shunt 20 closes or blocks cooling fluid space 82 toward the insertion end 85 of the outer conductive sleeve 18.

Shunt 20 extends beyond the actual end 86 of the outer conductor 27 to form an enlarged inside diameter shunt portion 87. The insertion end of enlarged diameter shunt portion 87 can accept a reduced diameter mounting portion 88 of the applicator tip 16 with dielectric collar 22 thereon. Dielectric collar 22 fits over the reduced diameter mounting portion 88 of the applicator tip 16, and itself has a reduced diameter insertion portion 89 that fits into enlarged inside diameter shunt portion 87. This interfitting arrangement produces a strong connection of the tip 16 to the remainder of the applicator, with the dielectric collar 22 being bonded to the tip and the shunt by an adhesive material such as, for example, epoxy.

Dielectric collar 22, being positioned between shunt 20 and tip 16, electrically insulates tip 16 from shunt 20. Because shunt 20 is electrically connected to the outer conductor 27 of the coaxial transmission line 26, shunt 20 becomes an extension of the outer conductor 27 and the insertion end 90 of the conductive shunt 20 becomes the effective insertion end of the outer conductor 27. The inner conductor 29 of the coaxial transmission line extends toward the insertion end of the applicator beyond the insertion end 91 of the coaxial transmission line dielectric material 28 to an inner conductor insertion end 92. However, both the insertion end 91 of the coaxial transmission line dielectric material and the insertion end 92 of the coaxial transmission line inner conductor are within the enlarged inside diameter shunt portion 87 of shunt 20 and do not extend beyond the insertion end 90 of shunt 20.

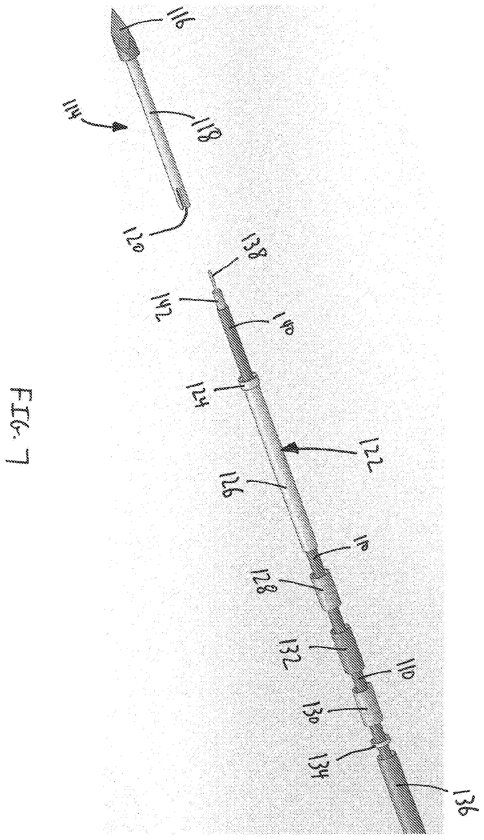

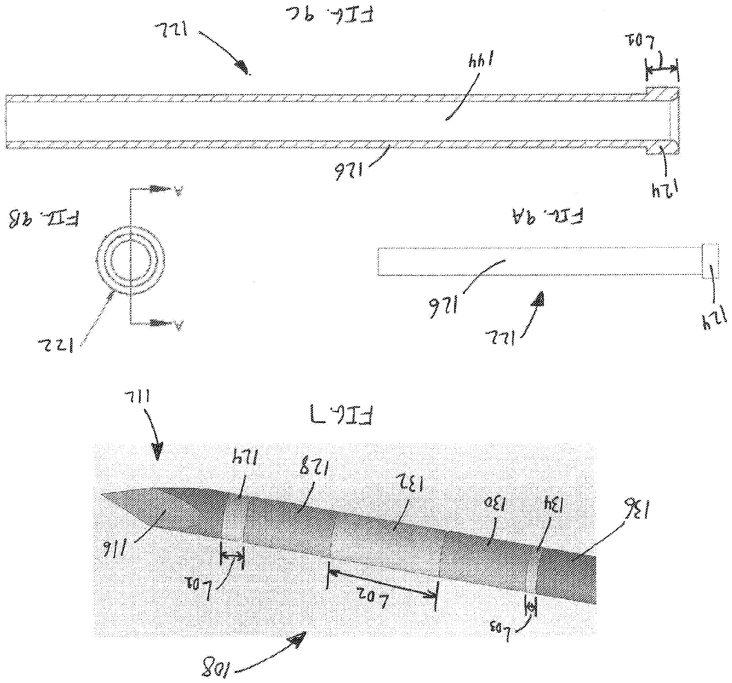

The reduced diameter mounting portion 88 of applicator tip 16 also includes a tip tab 93 extending therefrom toward the handle end of the applicator and the insertion end 91 of the coaxial transmission line dielectric 28. The tip tab 93 is positioned so that the extension of the coaxial transmission line inner conductor 29 beyond the end 91 of the coaxial transmission line dielectric 28 is adjacent to and can be secured in electrical contact, such as by soldering, to the tip tab 93. With this arrangement, inner conductor 29 does not extend into tip 16, but is merely adjacent to and electrically connected to tip tab 93. In some embodiments, instead of having a tip tab, the mounting portion 88 of the applicator tip 16 can include a hole therein or can be slotted such as, for example, as depicted in FIG. 7, in order to receive the inner conductor 29 therein. A slotted construction facilitates easy access for bonding the inner conductor 29 to the applicator tip 16 either by soldering or by using a conductive bonding agent.

As constructed, the conductive outer sleeve 18 may be of a metal material such as stainless steel, the conductive tip and the shunt may be formed of a metal material such as, for example, brass or stainless steel, and the dielectric insulating collar 22 may be formed of a substantially rigid plastic material. All such parts may be bonded using an epoxy or other adhesive. Further, while the construction described for this illustrated embodiment provides an embodiment of a microwave antenna toward the insertion end of the applicator, various other applicator constructions can be used to form a microwave antenna toward the insertion end of the antenna and to form an insertion end of the applicator.

As seen in FIG. 2, a guide sleeve 40 extends into cooling fluid space 82 between the outside of coaxial transmission line 26 and the inside of outer conductive sleeve 18. Guide sleeve 40 divides cooling fluid space 82 into an inner cooling fluid space 42 and an outer cooling fluid space 43 along the length of guide sleeve 40 in space 82. Inner cooling fluid space 42 is formed between the outside surface of coaxial transmission line 26 and the inside surface of guide sleeve 40 and outer cooling fluid space 43 is formed between the outside surface of guide sleeve 40 and the inside surface of outer conductive sleeve 18. Thus, cooling fluid can be flowed into the elongate applicator body 14 through inner cooling space 42 along the outside surface of coaxial transmission line 26 to cool the outside surface of coaxial transmission line 26. As previously indicated in regard to FIG. 2, cooling fluid space 82 into which guide sleeve 40 extends is blocked at the insertion end portion of outer conductive sleeve 18 by the reduced diameter portion 84 of shunt 20, which fits into and blocks the insertion end of space 82. As seen in FIG. 2, the insertion end 41 of guide sleeve 40 ends before reaching the end of space 82 created by shunt 20 so as to leave an undivided fluid space portion that connects the inner cooling fluid space 42 and the outer cooling fluid space 43. Thus, as cooling fluid flowing in inner cooling fluid space 42 toward the insertion end of the applicator reaches the insertion end 41 of guide sleeve 40, it flows into the undivided space 82 around the insertion end 41 of guide sleeve 40 into outer cooling fluid space 43 and flows along the inside surface of outer conductive sleeve 18 and exits the elongate applicator body 14 at the handle 12. As will be understood by those of skill in the art, the cooling fluid can also be flowed into the elongate applicator body 14 through outer cooling space 43, into the undivided space 82 around the insertion end 41 of guide sleeve 40, into inner cooling fluid space 42 and exits the elongate applicator body at the handle 12.

The handle 12 and cooling supply system can be constructed as disclosed and described in the following commonly-assigned U.S. patents: U.S. Patent Application No. 12/620,002, which issued as U.S. Patent No. 8,414,570 on April 9, 2013, entitled "MICROWAVE COAGULATION APPLICATOR AND SYSTEM," filed November 17, 2009 by Paul F. Turner et al.; and U.S. Patent Application No. 12/689,195, which issued as U.S. Patent No. 8,551,083 on October 8, 2013, entitled "MICROWAVE COAGULATION APPLICATOR AND SYSTEM," filed January 18, 2010 by Paul F. Turner et al

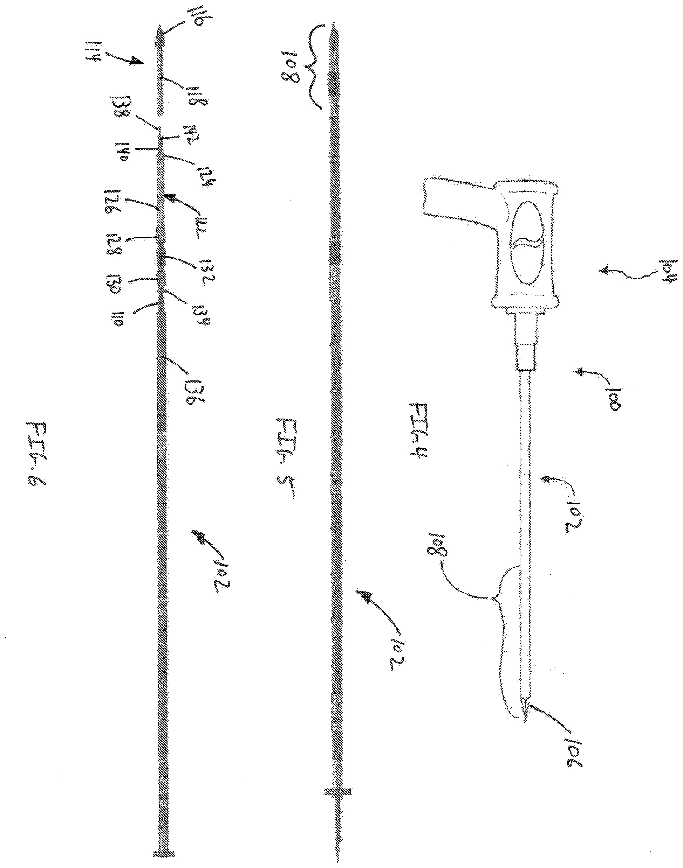

Depicted in FIGS. 4-12 is another embodiment of an applicator 100 having an elongate applicator body 102. The applicator 100 includes a handle 104 from which the elongate applicator body 102 extends with an insertion tip 106 forming the insertion end portion of the applicator for insertion into a region of the target tissue to be ablated. In some embodiments, the elongate applicator body 102 is substantially rigid. The elongate applicator body 102 has a microwave antenna portion 108 toward the insertion tip 106 to radiate microwave energy from the antenna portion into the target tissue. Microwave energy is transmitted from the handle 104 through the elongate applicator body 102 to the antenna portion 108 by a coaxial microwave transmission line 110, within the elongate applicator body 102.

This embodiment is directed to achieving a more spherical ablation zone as a result of a more spherical microwave energy field emission from the microwave antenna portion 108. As disclosed below, by changing/adjusting the lengths of the plurality of dielectric isolators and hence, the graded window of the dielectrics in the microwave antenna portion 108, one can change the size and shape of the microwave energy field being emitted from the antenna portion 108 (i.e., one can utilize near field beam shaping to change the near field microwave energy emission (microwave beam) to achieve desired sphericities of the ablation zone).

As depicted in FIGS. 5-8, the elongate applicator body 102, includes a microwave antenna portion 108 at the insertion end 112 of the applicator body 102. The antenna portion 108 includes a tip portion 114 having a trocar tip 116 for piercing/penetrating tissue and an elongate mounting portion 118. As can be seen in FIG. 8, in some embodiments, the end 120 of the elongate mounting portion 118 opposite the trocar tip 116, can be slotted or can have a central hole therein to receive the inner/center conductor of the coaxial microwave transmission line 110 (as described below). In some embodiments, the tip portion 114 can be made from a conductive metal material such as, for example, brass or stainless steel.

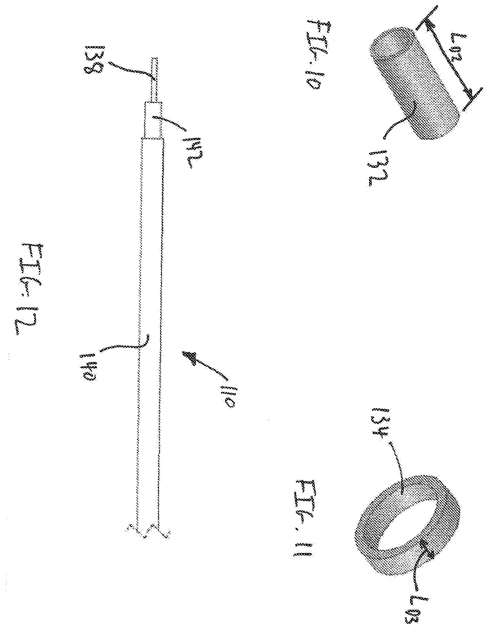

The microwave antenna portion 108 also includes: a first dielectric isolator 122 having a larger diameter cylindrical portion 124 with a width/length L_{D1} and an elongated reduced diameter cylindrical portion 126 (see FIGS. 9A-9C); a first shunt 128, a second shunt 130, a second dielectric isolator 132 having a width/length L_{D2} (see FIG. 10) disposed between the first shunt 128 and the second shunt 130, a third dielectric isolator 134 having a width/length L_{D3} (see FIG. 11) and a hypotube or outer sleeve 136, which may be, for example, stainless steel. Included on the interior of the microwave antenna portion 108 is a coaxial microwave transmission line 110. As can best be seen in FIG. 12, the coaxial microwave transmission line 110 includes a center or inner conductor 138, an outer conductor 140 and a dielectric isolator 142 surrounding the inner conductor 138 and disposed between the inner conductor 138 and outer conductor 140. Because three (3) dielectric isolators are used, this embodiment has a three (3) slot microwave antenna portion 108.

As can be seen in the figures, in this embodiment, the first dielectric isolator 122, the first shunt 128, the second shunt 130, the second dielectric isolator 132 and the third dielectric isolator 134 are all in the form of a cylinder/tube with an internal longitudinal lumen therethrough. Therefore, the microwave antenna portion 108 can be constructed by, for example, placing the first shunt 128, the second dielectric isolator 132, the second shunt 130 and the third dielectric isolator 134 onto the elongated reduced diameter cylindrical portion 126 of the first dielectric isolator 122. With the components assembled on the elongated reduced diameter cylindrical portion 126 of the first dielectric isolator 122, the coaxial microwave transmission line 110 can be inserted into the internal longitudinal lumen 144 (FIG. 9C) of the first dielectric isolator 122. The inner conductor 138 can then be inserted into the end 120 of the elongate mounting portion 118 of the tip portion 114 and bonded thereto either by soldering, conductive epoxy or by any other conductive bonding means known in the art. With the inner conductor 138 bonded to the tip portion 114, the first dielectric isolator 122 can be slid onto the elongate mounting portion 118 of the tip portion 114 and the components bonded in place as required using any bonding means such as, for example, epoxy. Depicted in FIG. 8 is a fully assembled microwave antenna portion 108 according to an embodiment of the invention. Although not shown in the figures, the microwave antenna portion 108 can include a cooling system with supply and return spaces similar to that disclosed in the previous embodiment.

In some embodiments, the first dielectric isolator 122, the second dielectric isolator 132 and the third dielectric isolator 134 are made of polyether ether ketone (PEEK) and the first shunt 128 and second shunt 130 are made of brass. As will be understood by those of skill in the art other dielectric materials may be used for the isolators and other materials may be used for the shunts such as, for example, stainless steel. In some embodiments, the trocar tip 116, isolators 124, 132, 134, and shunts 128, 130, may include a coating. In some embodiments, the isolators may be coated with fluorobond N-2 and the trocar tip and shunts may be coated with polytetrafluoroethylene (PTFE).

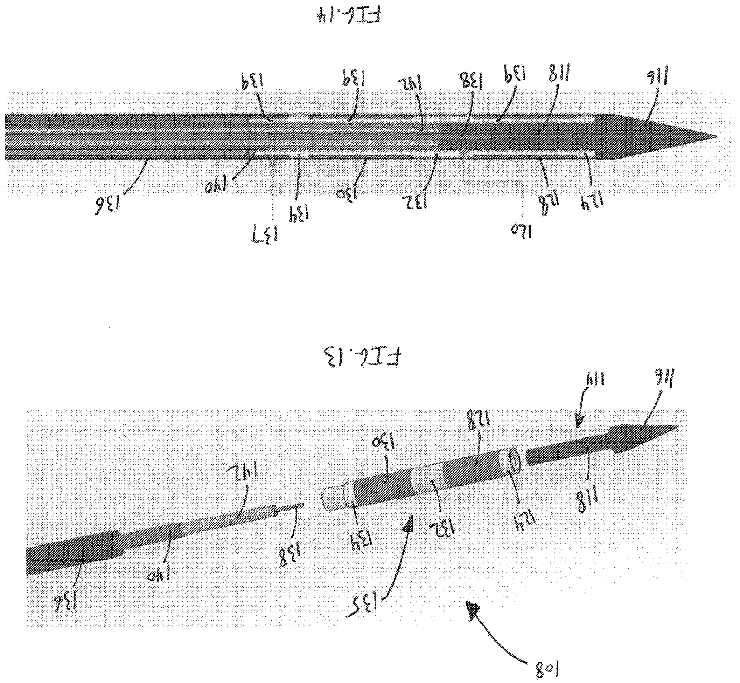

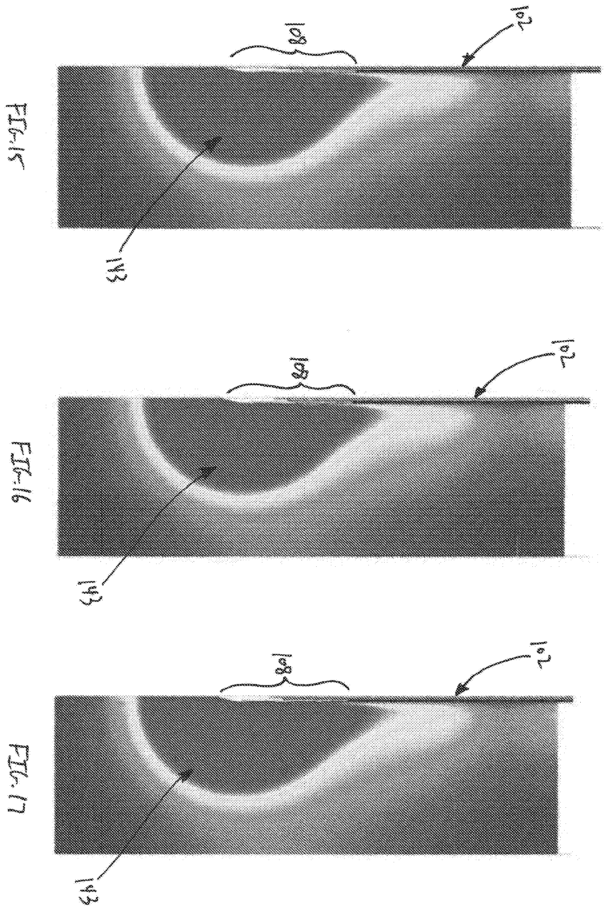

The embodiment depicted in FIGS. 13 and 14 has similar components to the embodiment depicted in FIGS. 5-8 but differs in construction. Instead of using three individual components for the three dielectric isolators, this embodiment uses a liquid crystal polymer insert molded monolithic dielectric isolator 139. That is, the dielectric isolators 124, 132, 134 are molded as a single component with the first shunt 128 and second shunt 130 molded thereon. Thus, the dielectric isolators 124, 132, 134 and first shunt 128 and second shunt 130 are essentially a single component 135 for the assembly process. In this embodiment, as can be seen in FIG. 15, the monolithic dielectric isolator 139 includes a first major cylindrical portion 124 with a first major isolator diameter and a first major isolator width (LD1), a first minor cylindrical portion with a first minor isolator diameter less than the first major isolator diameter and a first minor isolator width, a second major cylindrical portion 132 with a second major isolator diameter and a second major isolator width (LD2), a second minor cylindrical portion with a second minor isolator diameter less than the second major isolator diameter and a second minor isolator width, and a third major cylindrical portion 134 with a third major isolator diameter greater than the second minor isolator diameter and a third major isolator width (LD3). Further, the first cylindrical shunt 128 is disposed on the first minor cylindrical portion and the second cylindrical shunt 130 is disposed on the second minor cylindrical portion. Also, as can best be seen in FIG. 14, the outer conductor 140 is removed from the coaxial microwave transmission line 110 to point 137, which is just beyond dielectric isolator 134. Accordingly, a single slot is created in the coaxial microwave transmission line but multiple slots (3) are created in the single component by virtue of the three dielectric isolators 124, 132, 134.

As previously disclosed, changing the lengths L_{D1}, L_{D2} and L_{D3} of the dielectric isolators 124, 132, 134 (see FIG. 8), changes the shape and/or sphericity of the microwave energy field emitted from the microwave antenna portion 108. For Example 1, a microwave antenna portion 108 with three (3) slots was constructed with dielectric isolators 124, 132, 134 having the following lengths, respectively: L_{D1} = 1.0 mm, L_{D2} = 5.0 mm and L_{D3} = 0.5 mm. Depicted in FIG. 15 is the temperature profile 143 of the microwave energy field of an applicator with the described constructed applicator body powered at 915 MHz. For Example 2, a microwave antenna portion 108 with a three (3) slots was constructed with dielectric isolators 124, 132, 134 having the following lengths, respectively: L_{D1} = 1.0 mm, L_{D2} = 3.0 mm and L_{D3} = 0.5 mm. Depicted in FIG. 16 is the temperature profile 143 of the microwave energy field of an applicator with the described constructed applicator body powered at 915 MHz. For Example 3, a microwave antenna portion 108 with a three (3) slots was constructed with dielectric isolators 124, 132, 134 having the following lengths, respectively: L_{D1} = 1.0 mm, L_{D2} = 3.0 mm and L_{D3} = 1.0 mm. Depicted in FIG. 17 is the temperature profile 143 of the microwave energy field of an applicator with the described constructed applicator body powered at 915 MHz. As depicted in FIGS. 15-17, while it may be difficult to see, the sphericity of the temperature profile of the microwave energy field in each example differs. Therefore, the sphericity of the microwave energy field can be fine-tuned by changing the lengths/widths of the dielectric isolators 124, 132, 134. Based on testing, the construction that provides the optimized microwave energy filed appears to be the construction with dielectric isolators 124, 132, 134 having the following lengths, respectively: L_{D1} = 1.0 mm, L_{D2} = 5.0 mm and L_{D3} = 0.5 mm.

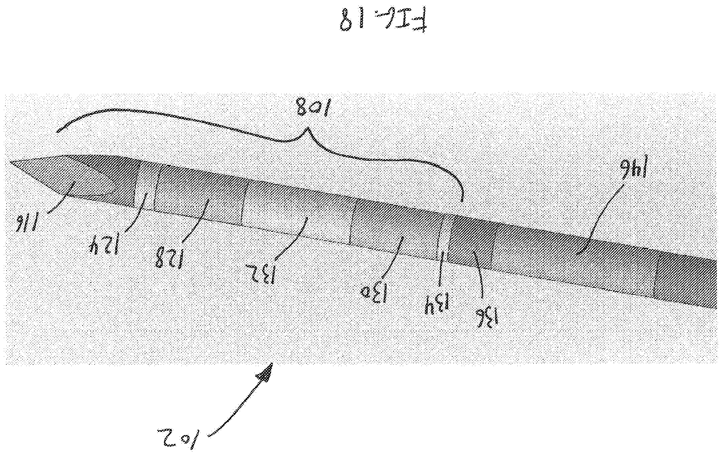

In some embodiments, a magnetic choke or "magnetic firewall" can be included. The magnetic choke, in this implementation, provides a "firewall" blocking the ablation pattern from extending proximally along the elongate body of the probe away from the insertion tip. This can be achieved by adding a magnetic sleeve 146 made from a highly-permeable magnetic metal, such as, for example, permalloy, to the elongate applicator body 102 as depicted in FIG. 18. Including a magnetic sleeve 146 on the elongate applicator body 102 acts to prevent the microwave energy field from extending up the elongate applicator body 102 toward the tissue insertion site and the handle, thereby better controlling the return path of the microwave energy field. Including a magnetic sleeve 146 on the elongate applicator body 102 also enables one to move the microwave energy field farther down the elongate applicator body 102 towards the trocar tip 116 further increasing/improving the sphericity of the microwave energy field. Moving the magnetic sleeve 146 closer to the trocar tip 116 can also result in further improvements to the precision and accuracy of how far beyond the trocar tip 116 the microwave energy field extends beyond the tip 116 and penetrates into the tissue.

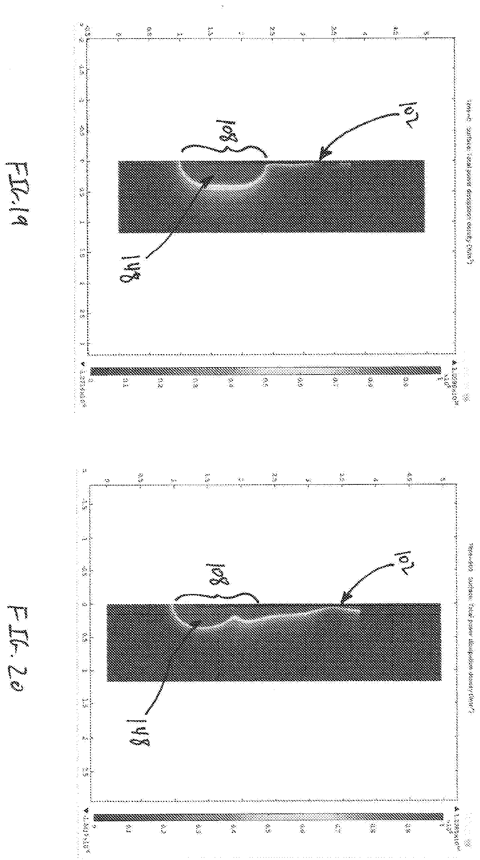

To demonstrate the effect of including a magnetic sleeve 146 on the elongate applicator body, a first elongate applicator body 102 having a microwave antenna portion 108 with a three (3) slots as depicted in FIGS. 8 and 18 was constructed with dielectric isolators 124, 132, 134 having the following lengths, respectively: L_{D1} = 1.0 mm, L_{D2} = 5.0 mm and L_{D3} = 0.5 mm. A magnetic sleeve 146 was included on the elongate applicator body 102 above the microwave antenna portion 108 adjacent to the third dielectric isolator 134 provides a magnetic choke. Depicted in FIG. 19 is the temperature profile 148 of the microwave energy field of an applicator with the described constructed applicator body powered at 915 MHz. A second elongate applicator body was constructed with a microwave antenna portion 108 similar to the first elongate applicator body except that a magnetic sleeve 146 was not included. Depicted in FIG. 20 is the temperature profile 148 of the microwave energy field of an applicator with the described constructed applicator body powered at 915 MHz. As can be seen in FIGS. 19 and 20, the magnetic sleeve 146 prevented the microwave energy field from extending up the elongate applicator body 102. Therefore, the microwave energy field is concentrated at the desired location near the trocar tip 116 of the elongate applicator body 102, which results in a more powerful microwave antenna, which can lead to shorter procedure times.

In order to prevent sticking to body tissue, the elongate applicator body may be coated with a stick resistant dielectric material such as, for example, Teflon.

Many modifications and variations of the present invention are possible in light of the above teachings. It is therefore to be understood that within the scope of the appended claims the invention may be practiced otherwise than as specifically described.

## Claims

1. An elongate applicator body for use with a microwave applicator, the elongate applicator body comprising:
a tip portion (114) including a tip elongate portion;
a first dielectric isolator (122) having a first dielectric width and a longitudinal lumen, L_{DI};
a first cylindrical shunt (128);
a second cylindrical dielectric isolator (132) having a second dielectric width, L_{D2};
a second cylindrical shunt (130);
a third cylindrical dielectric isolator (134) having a third dielectric width, L_{D3}; and
a microwave transmission line (110);
**characterized in that**:
the first dielectric isolator comprises a first cylindrical portion with a first isolator first diameter, and an elongated second cylindrical portion with a first isolator second diameter that is less than the first isolator first diameter;
the first cylindrical shunt, the second cylindrical dielectric isolator, the second cylindrical shunt and the third cylindrical dielectric isolator are disposed on the elongated second cylindrical portion of the first dielectric isolator;
at least a portion of the tip elongate portion is disposed within the longitudinal lumen of the first dielectric isolator;
at least a portion of the microwave transmission line is also disposed within a portion of the longitudinal lumen of the first dielectric isolator; and
an inner conductor of the microwave transmission line is received within an end of the tip elongate portion.

2. The elongate applicator body of claim 1, wherein the tip portion comprises a trocar tip.

3. The elongate applicator body of claim 1 or 2, wherein L_{DI}< L_{D2}> L_{D3}.

4. The elongate applicator body of claim 3, wherein:
L_{DI}= 1.0 mm;
L_{D2}= 5.0 mm; and
L_{D3}= 0.5 mm.

5. The elongate applicator body of any one of the preceding claims, wherein the first dielectric isolator, the second dielectric isolator and the third dielectric isolator comprise polyether ether ketone, PEEK.

6. The elongate applicator body of any one of the preceding claims, wherein the first shunt and the second shunt comprise brass.

7. The elongate applicator body of any one of the preceding claims, wherein the first dielectric isolator, the second dielectric isolator and the third dielectric isolator are individual components.

8. The elongate applicator body of any one of the preceding claims, wherein the first dielectric isolator, the second dielectric isolator and the third dielectric isolator are a single monolithic structure.

9. The elongate applicator body of any one of the preceding claims, further comprising a magnetic sleeve.

10. The elongate applicator body of any one of the preceding claims, wherein:
the first cylindrical shunt has a first shunt diameter and a first shunt width;
the second cylindrical dielectric isolator has a second isolator diameter;
the second cylindrical shunt has a second shunt diameter and a second shunt width;
the third cylindrical dielectric isolator has a third isolator diameter.

11. The elongate applicator body of any one of the preceding claims, including an outer sleeve (18).

12. The elongate applicator body of any preceding claim, wherein:
the first cylindrical portion of the first cylindrical dielectric isolator is adjacent to a or the trocar tip,
the first cylindrical shunt is adjacent to the first cylindrical portion, the second cylindrical dielectric isolator is adjacent to the first cylindrical shunt,
the second cylindrical shunt is adjacent to the second cylindrical dielectric isolator, and
the third cylindrical dielectric isolator is adjacent to the second cylindrical shunt.

13. A microwave applicator for insertion into body tissue, the microwave applicator comprising:
an elongate applicator body according to claim 11 and having an insertion end for insertion into a tissue region of the living body and an attachment end for attachment to a source of microwave energy, wherein the microwave energy transmission line is disposed within the elongate applicator body to conduct microwave energy from the attachment end of the applicator to the insertion end, the microwave energy transmission line having an inner conductor and an outer conductor, and the outer sleeve extends around and is spaced from the outer conductor of the microwave energy transmission line to form an outside of a portion of the elongate applicator body and to provide a cooling fluid space between the outer conductor of the microwave transmission line and an inside surface of the outer sleeve; and
a guide sleeve positioned concentrically around and spaced from the outer conductor of the microwave energy transmission line and the inside surface of the outer sleeve to divide at least a portion of the cooling fluid space into an inner cooling fluid space along the outer conductor of the microwave energy transmission line and an outer cooling fluid space along the inside surface of the outer sleeve, the inner cooling fluid space communicating with the outer cooling fluid space wherein the guide sleeve is adapted to guide flow of a cooling fluid within the cooling fluid space to cool the microwave energy transmission line and the outer sleeve.

14. The microwave applicator for insertion into body tissue according to claim 13, further comprising a handle from which the outer conductive sleeve, microwave energy transmission line, and the guide sleeve extend.

15. The microwave applicator for insertion into body tissue according to claim 14, wherein the handle includes an inlet for connection to a source of cooling fluid and an outlet for outflow of cooling fluid, and wherein, when cooling fluid is supplied through the cooling fluid inlet, the cooling fluid flows into the cooling fluid space in the elongate applicator body from the handle and returns from the cooling fluid space in the elongate applicator body into the handle.

16. The microwave applicator for insertion into body tissue according to claim 15, wherein, when cooling fluid is supplied through the cooling fluid inlet:
the cooling fluid flows from the handle into the inner cooling fluid space and returns to the handle through the outer cooling fluid space; or
the cooling fluid flows from the handle into the outer cooling fluid space and returns to the handle through the inner cooling fluid space.

17. A microwave applicator for insertion into body tissue, the microwave applicator comprising:
an elongate applicator body according to claims 10 and 11, wherein the microwave energy transmission line is disposed within the elongate applicator body to conduct microwave energy from the attachment end of the applicator to the insertion end, the microwave energy transmission line having an inner conductor and an outer conductor, and the outer sleeve extends around and is spaced from the outer conductor of the microwave energy transmission line to form an outside of a portion of the elongate applicator body and to provide a cooling fluid space between the outer conductor of the microwave transmission line and an inside surface of the outer sleeve; and
a guide sleeve positioned concentrically around and spaced from the outer conductor of the microwave energy transmission line and the inside surface of the outer sleeve to divide at least a portion of the cooling fluid space into an inner cooling fluid space along the outer conductor of the microwave energy transmission line and an outer cooling fluid space along the inside surface of the outer sleeve, the inner cooling fluid space communicating with the outer cooling fluid space wherein the guide sleeve is adapted to guide flow of a cooling fluid within the cooling fluid space to cool the microwave energy transmission line and the outer sleeve.

## Patentansprüche

1. Länglicher Applikatorkörper zur Verwendung mit einem Mikrowellenapplikator, wobei der längliche Applikatorkörper Folgendes umfasst:
einen Spitzenabschnitt (114), der einen länglichen Spitzenabschnitt beinhaltet;
einen ersten dielektrischen Isolator (122), der eine erste dielektrische Breite und ein Längslumen L_{DI} aufweist;
einen ersten zylindrischen Shunt (128);
einen zweiten zylindrischen dielektrischen Isolator (132), der eine zweite dielektrische Breite L_{D2} aufweist;
einen zweiten zylindrischen Shunt (130);
einen dritten zylindrischen dielektrischen Isolator (134), der eine dritte dielektrische Breite L_{D3} aufweist; und
eine Mikrowellenübertragungsleitung (110);
**dadurch gekennzeichnet, dass**:
der erste dielektrische Isolator einen ersten zylindrischen Abschnitt mit einem ersten Durchmesser des ersten Isolators und einen langgestreckten zweiten zylindrischen Abschnitt mit einem zweiten Durchmesser des ersten Isolators, der geringer als der erste Durchmesser des ersten Isolators ist, umfasst;
der erste zylindrische Shunt, der zweite zylindrische dielektrische Isolator, der zweite zylindrische Shunt und der dritte zylindrische dielektrische Isolator an dem langgestreckten zweiten zylindrischen Abschnitt des ersten dielektrischen Isolators angeordnet sind;
mindestens ein Abschnitt des länglichen Spitzenabschnitts innerhalb des Längslumens des ersten dielektrischen Isolators angeordnet ist;
mindestens ein Abschnitt der Mikrowellenübertragungsleitung ebenfalls innerhalb eines Abschnitts des Längslumens des ersten dielektrischen Isolators angeordnet ist; und
ein innerer Leiter der Mikrowellenübertragungsleitung in einem Ende des länglichen Spitzenabschnitts aufgenommen ist.

2. Länglicher Applikatorkörper nach Anspruch 1, wobei der Spitzenabschnitt eine Trokarspitze umfasst.

3. Länglicher Applikatorkörper nach Anspruch 1 oder 2, wobei L_{DI}< L_{D2}> L_{D3}.

4. Länglicher Applikatorkörper nach Anspruch 3, wobei:
L_{DI}= 1, 0 mm;
L_{D2}= 5, 0 mm; und
L_{D3}= 0, 5 mm.

5. Länglicher Applikatorkörper nach einem der vorhergehenden Ansprüche, wobei der erste dielektrische Isolator, der zweite dielektrische Isolator und der dritte dielektrische Isolator Polyetheretherketon (PEEK) umfassen.

6. Länglicher Applikatorkörper nach einem der vorhergehenden Ansprüche, wobei der erste Shunt und der zweite Shunt Messing umfassen.

7. Länglicher Applikatorkörper nach einem der vorhergehenden Ansprüche, wobei der erste dielektrische Isolator, der zweite dielektrische Isolator und der dritte dielektrische Isolator einzelne Komponenten sind.

8. Länglicher Applikatorkörper nach einem der vorhergehenden Ansprüche, wobei der erste dielektrische Isolator, der zweite dielektrische Isolator und der dritte dielektrische Isolator eine einzige monolithische Struktur sind.

9. Länglicher Applikatorkörper nach einem der vorhergehenden Ansprüche, ferner umfassend eine magnetische Hülse.

10. Länglicher Applikatorkörper nach einem der vorhergehenden Ansprüche, wobei:
der erste zylindrische Shunt einen ersten Shuntdurchmesser und eine erste Shuntbreite aufweist;
der zweite zylindrische dielektrische Isolator einen zweiten Isolatordurchmesser aufweist;
der zweite zylindrische Shunt einen zweiten Shuntdurchmesser und eine zweite Shuntbreite aufweist;
der dritte zylindrische dielektrische Isolator einen dritten Isolatordurchmesser aufweist.

11. Länglicher Applikatorkörper nach einem der vorhergehenden Ansprüche, der eine äußere Hülse (18) beinhaltet.

12. Länglicher Applikatorkörper nach einem der vorhergehenden Ansprüche, wobei:
der erste zylindrische Abschnitt des ersten zylindrischen dielektrischen Isolators an eine oder die Trokarspitze angrenzt,
der erste zylindrische Shunt an den ersten zylindrischen Abschnitt angrenzt, der zweite zylindrische dielektrische Isolator an den ersten zylindrischen Shunt angrenzt,
der zweite zylindrische Shunt an den zweiten zylindrischen dielektrischen Isolator angrenzt und
der dritte zylindrische dielektrische Isolator an den zweiten zylindrischen Shunt angrenzt.

13. Mikrowellenapplikator zum Einführen in Körpergewebe, wobei der Mikrowellenapplikator Folgendes umfasst:
einen länglichen Applikatorkörper nach Anspruch 11, der ein Einführende zum Einführen in einen Gewebebereich des lebenden Körpers und ein Anbringende zum Anbringen an einer Mikrowellenenergiequelle aufweist, wobei die Mikrowellenenergieübertragungsleitung innerhalb des länglichen Applikatorkörpers angeordnet ist, um Mikrowellenenergie von dem Anbringende des Applikators zu dem Einführende zu leiten, wobei die Mikrowellenenergieübertragungsleitung einen inneren Leiter und einen äußeren Leiter aufweist und sich die äußere Hülse um den äußeren Leiter der Mikrowellenenergieübertragungsleitung erstreckt und von diesem beabstandet ist, um eine Außenseite eines Abschnitts des länglichen Applikatorkörpers zu bilden und einen Kühlflüssigkeitsraum zwischen dem äußeren Leiter der Mikrowellenübertragungsleitung und einer Innenfläche der äußeren Hülse bereitzustellen; und
eine Führungshülse, die konzentrisch um den äußeren Leiter der Mikrowellenenergieübertragungsleitung und die Innenfläche der äußeren Hülse positioniert und von diesen beabstandet ist, um mindestens einen Abschnitt des Kühlflüssigkeitsraums in einen inneren Kühlflüssigkeitsraum entlang des äußeren Leiters der Mikrowellenenergieübertragungsleitung und einen äußeren Kühlflüssigkeitsraum entlang der Innenfläche der äußeren Hülse zu unterteilen, wobei der innere Kühlflüssigkeitsraum mit dem äußeren Kühlflüssigkeitsraum in Verbindung steht, wobei die Führungshülse dazu angepasst ist, den Strom einer Kühlflüssigkeit innerhalb des Kühlflüssigkeitsraums zu führen, um die Mikrowellenenergieübertragungsleitung und die äußere Hülse zu kühlen.

14. Mikrowellenapplikator zum Einführen in Körpergewebe nach Anspruch 13, ferner umfassend einen Griff, von dem sich die äußere leitfähige Hülse, die Mikrowellenenergieübertragungsleitung und die Führungshülse erstrecken.

15. Mikrowellenapplikator zum Einführen in Körpergewebe nach Anspruch 14, wobei der Griff einen Einlass zum Anschluss an eine Quelle für Kühlflüssigkeit und einen Auslass zum Ausströmen von Kühlflüssigkeit beinhaltet und wobei, wenn Kühlflüssigkeit durch den Kühlflüssigkeitseinlass zugeführt wird, die Kühlflüssigkeit von dem Griff in den Kühlflüssigkeitsraum in dem länglichen Applikatorkörper strömt und von dem Kühlflüssigkeitsraum in dem länglichen Applikatorkörper in den Griff zurückkehrt.

16. Mikrowellenapplikator zum Einführen in Körpergewebe nach Anspruch 15, wobei, wenn Kühlflüssigkeit durch den Kühlflüssigkeitseinlass zugeführt wird:
die Kühlflüssigkeit von dem Griff in den inneren Kühlflüssigkeitsraum strömt und durch den äußeren Kühlflüssigkeitsraum zu dem Griff zurückkehrt; oder
die Kühlflüssigkeit von dem Griff in den äußeren Kühlflüssigkeitsraum strömt und durch den inneren Kühlflüssigkeitsraum zu dem Griff zurückkehrt.

17. Mikrowellenapplikator zum Einführen in Körpergewebe, wobei der Mikrowellenapplikator Folgendes umfasst:
einen länglichen Applikatorkörper nach Anspruch 10 und 11, wobei die Mikrowellenenergieübertragungsleitung innerhalb des länglichen Applikatorkörpers angeordnet ist, um Mikrowellenenergie von dem Anbringende des Applikators zu dem Einführende zu leiten, wobei die Mikrowellenenergieübertragungsleitung einen inneren Leiter und einen äußeren Leiter aufweist und sich die äußere Hülse um den äußeren Leiter der Mikrowellenenergieübertragungsleitung erstreckt und von diesem beabstandet ist, um eine Außenseite eines Abschnitts des länglichen Applikatorkörpers zu bilden und einen Kühlflüssigkeitsraum zwischen dem äußeren Leiter der Mikrowellenübertragungsleitung und einer Innenfläche der äußeren Hülse bereitzustellen; und
eine Führungshülse, die konzentrisch um den äußeren Leiter der Mikrowellenenergieübertragungsleitung und die Innenfläche der äußeren Hülse positioniert und von diesen beabstandet ist, um mindestens einen Abschnitt des Kühlflüssigkeitsraums in einen inneren Kühlflüssigkeitsraum entlang des äußeren Leiters der Mikrowellenenergieübertragungsleitung und einen äußeren Kühlflüssigkeitsraum entlang der Innenfläche der äußeren Hülse zu unterteilen, wobei der innere Kühlflüssigkeitsraum mit dem äußeren Kühlflüssigkeitsraum in Verbindung steht, wobei die Führungshülse dazu angepasst ist, den Strom einer Kühlflüssigkeit innerhalb des Kühlflüssigkeitsraums zu führen, um die Mikrowellenenergieübertragungsleitung und die äußere Hülse zu kühlen.

## Revendications

1. Corps d'applicateur allongé destiné à être utilisé avec un applicateur à micro-ondes, le corps d'applicateur allongé comprenant :
une partie pointe (114) comportant une partie pointe allongée ;
un premier isolateur diélectrique (122) ayant une première largeur diélectrique et une lumière longitudinale, L_{DI} ;
un premier shunt cylindrique (128) ;
un deuxième isolateur diélectrique cylindrique (132) ayant une deuxième largeur diélectrique, L_{D2} ;
un second shunt cylindrique (130) ;
un troisième isolateur diélectrique cylindrique (134) ayant une troisième largeur diélectrique, L_{D3} ; et
une ligne de transmission micro-ondes (110) ;
**caractérisé en ce que** :
le premier isolateur diélectrique comprend une première partie cylindrique avec un premier diamètre de premier isolateur, et une seconde partie cylindrique allongée avec un deuxième diamètre de premier isolateur qui est inférieur au premier diamètre de premier isolateur ;
le premier shunt cylindrique, le deuxième isolateur diélectrique cylindrique, le second shunt cylindrique et le troisième isolateur diélectrique cylindrique sont disposés sur la seconde partie cylindrique allongée du premier isolateur diélectrique ;
au moins une partie de la partie pointe allongée est disposée à l'intérieur de la lumière longitudinale du premier isolateur diélectrique ;
au moins une partie de la ligne de transmission micro-ondes est également disposée à l'intérieur d'une partie de la lumière longitudinale du premier isolateur diélectrique ; et
un conducteur interne de la ligne de transmission micro-ondes est reçu à l'intérieur d'une extrémité de la partie pointe allongée.

2. Corps d'applicateur allongé selon la revendication 1, dans lequel la partie pointe comprend une pointe de trocart.

3. Corps d'applicateur allongé selon la revendication 1 ou 2, dans lequel L_{DI} < L_{D2} > L_{D3}.

4. Corps d'applicateur allongé selon la revendication 3, dans lequel :
L_{DI}= 1, 0 mm ;
L_{D2}= 5, 0 mm ; et
L_{D3}= 0,5 mm.

5. Corps d'applicateur allongé selon l'une quelconque des revendications précédentes, dans lequel le premier isolateur diélectrique, le deuxième isolateur diélectrique et le troisième isolateur diélectrique comprennent du polyéther éther cétone, PEEK.

6. Corps d'applicateur allongé selon l'une quelconque des revendications précédentes, dans lequel le premier shunt et le second shunt comprennent du laiton.

7. Corps d'applicateur allongé selon l'une quelconque des revendications précédentes, dans lequel le premier isolateur diélectrique, le deuxième isolateur diélectrique et le troisième isolateur diélectrique sont des composants individuels.

8. Corps d'applicateur allongé selon l'une quelconque des revendications précédentes, dans lequel le premier isolateur diélectrique, le deuxième isolateur diélectrique et le troisième isolateur diélectrique sont une structure monobloc.

9. Corps d'applicateur allongé selon l'une quelconque des revendications précédentes, comprenant en outre un manchon magnétique.

10. Corps d'applicateur allongé selon l'une quelconque des revendications précédentes, dans lequel :
le premier shunt cylindrique a un premier diamètre de shunt et une première largeur de shunt ;
le deuxième isolateur diélectrique cylindrique a un deuxième diamètre d'isolateur ;
le deuxième shunt cylindrique a un deuxième diamètre de shunt et une deuxième largeur de shunt ;
le troisième isolateur diélectrique cylindrique a un troisième diamètre d'isolateur.

11. Corps d'applicateur allongé selon l'une quelconque des revendications précédentes, comportant un manchon externe (18).

12. Corps d'applicateur allongé selon une quelconque revendication précédente, dans lequel :
la première partie cylindrique du premier isolateur diélectrique cylindrique est adjacente à une ou à la pointe de trocart,
le premier shunt cylindrique est adjacent à la première partie cylindrique, le deuxième isolateur diélectrique cylindrique est adjacent au premier shunt cylindrique,
le second shunt cylindrique est adjacent au deuxième isolateur diélectrique cylindrique, et
le troisième isolateur diélectrique cylindrique est adjacent au second shunt cylindrique.

13. Applicateur à micro-ondes destiné à être inséré dans un tissu corporel, l'applicateur à micro-ondes comprenant :
un corps d'applicateur allongé selon la revendication 11 et ayant une extrémité d'insertion destinée à être insérée dans une région tissulaire du corps vivant et une extrémité de fixation destinée à être fixée à une source d'énergie micro-ondes, dans lequel la ligne de transmission d'énergie micro-ondes est disposée à l'intérieur du corps d'applicateur allongé pour conduire l'énergie micro-ondes depuis l'extrémité de fixation de l'applicateur jusqu'à l'extrémité d'insertion, la ligne de transmission d'énergie micro-ondes ayant un conducteur interne et un conducteur externe, et le manchon externe s'étend autour du conducteur externe de la ligne de transmission d'énergie micro-ondes et est espacé de celui-ci pour former un extérieur d'une partie du corps d'applicateur allongé et pour fournir un espace de fluide de refroidissement entre le conducteur externe de la ligne de transmission de micro-ondes et une surface intérieure du manchon externe ; et
un manchon de guidage positionné de manière concentrique autour du conducteur externe de la ligne de transmission d'énergie micro-ondes et espacé de celui-ci et de la surface intérieure du manchon externe pour diviser au moins une partie de l'espace de fluide de refroidissement en un espace de fluide de refroidissement interne le long du conducteur externe de la ligne de transmission d'énergie micro-ondes et un espace de fluide de refroidissement externe le long de la surface intérieure du manchon externe, l'espace de fluide de refroidissement interne communiquant avec l'espace de fluide de refroidissement externe, dans lequel le manchon de guidage est conçu pour guider l'écoulement d'un fluide de refroidissement à l'intérieur de l'espace de fluide de refroidissement pour refroidir la ligne de transmission d'énergie micro-ondes et le manchon externe.

14. Applicateur à micro-ondes destiné à être inséré dans un tissu corporel selon la revendication 13, comprenant en outre une poignée à partir de laquelle s'étendent le manchon conducteur externe, la ligne de transmission d'énergie micro-ondes et le manchon de guidage.

15. Applicateur à micro-ondes destiné à être inséré dans un tissu corporel selon la revendication 14, dans lequel la poignée comporte une entrée pour la connexion à une source de fluide de refroidissement et une sortie pour l'écoulement de fluide de refroidissement, et dans lequel, lorsque le fluide de refroidissement est fourni à travers l'entrée de fluide de refroidissement, le fluide de refroidissement s'écoule dans l'espace de fluide de refroidissement dans le corps d'applicateur allongé à partir de la poignée et revient de l'espace de fluide de refroidissement dans le corps d'applicateur allongé dans la poignée.

16. Applicateur à micro-ondes destiné à être inséré dans un tissu corporel selon la revendication 15, dans lequel, lorsque le fluide de refroidissement est fourni à travers l'entrée de fluide de refroidissement :
le fluide de refroidissement s'écoule de la poignée dans l'espace de fluide de refroidissement interne et retourne à la poignée à travers l'espace de fluide de refroidissement externe ; ou
le fluide de refroidissement s'écoule de la poignée dans l'espace de fluide de refroidissement externe et retourne à la poignée à travers l'espace de fluide de refroidissement interne.

17. Applicateur à micro-ondes destiné à être inséré dans un tissu corporel, l'applicateur à micro-ondes comprenant :
un corps d'applicateur allongé selon les revendications 10 et 11, dans lequel la ligne de transmission d'énergie micro-ondes est disposée à l'intérieur du corps d'applicateur allongé pour conduire l'énergie micro-ondes depuis l'extrémité de fixation de l'applicateur jusqu'à l'extrémité d'insertion, la ligne de transmission d'énergie micro-ondes ayant un conducteur interne et un conducteur externe, et le manchon externe s'étend autour du conducteur externe de la ligne de transmission d'énergie micro-ondes et est espacé de celui-ci pour former un extérieur d'une partie du corps d'applicateur allongé et pour fournir un espace de fluide de refroidissement entre le conducteur externe de la ligne de transmission de micro-ondes et une surface intérieure du manchon externe ; et
un manchon de guidage positionné de manière concentrique autour du conducteur externe de la ligne de transmission d'énergie micro-ondes et espacé de celui-ci et de la surface intérieure du manchon externe pour diviser au moins une partie de l'espace de fluide de refroidissement en un espace de fluide de refroidissement interne le long du conducteur externe de la ligne de transmission d'énergie micro-ondes et un espace de fluide de refroidissement externe le long de la surface intérieure du manchon externe, l'espace de fluide de refroidissement interne communiquant avec l'espace de fluide de refroidissement externe, dans lequel le manchon de guidage est conçu pour guider l'écoulement d'un fluide de refroidissement à l'intérieur de l'espace de fluide de refroidissement pour refroidir la ligne de transmission d'énergie micro-ondes et le manchon externe.
